# EUROPEAN PATENT SPECIFICATION

(11) **EP 3 369 412 B1**
(45) Date of publication and mention of the grant of the patent: **24.02.2021**
(21) Application number: 16860215.9
(22) Date of filing: 26.10.2016
(51) Int. Cl.: A61K 8/02, A61K 8/88, A61K 8/891, A61K 8/81, A61K 8/25, A61Q 19/00, A61Q 1/00, A61K 8/19, A61K 8/72, A61Q 1/12

(54) **METHOD FOR PRODUCING PRESSED MAKE-UP POWDER**
VERFAHREN ZUR HERSTELLUNG VON GEPRESSTEM MAKE-UP-PUDER
PROCÉDÉ DE PRODUCTION DE POUDRE DE MAQUILLAGE COMPACTE

(30) Priority: 30.10.2015 KR 20150151453
(43) Date of publication of application: 05.09.2018
(73) Proprietor: Amorepacific Corporation, Seoul 04386 (KR)
(72) Inventor: ROH, Young Hea, Yongin-si Gyeonggi-do 17074 (KR); PARK, Se Jun, Yongin-si Gyeonggi-do 17074 (KR); KIM, Kyung Nam, Yongin-si Gyeonggi-do 17074 (KR)
(74) Representative: dompatent von Kreisler Selting Werner - Partnerschaft von Patent- und Rechtsanwälten mbB
(86) International application number: PCT/KR2016/012100
(87) International publication number: WO 2017/074032

(56) References cited:
- WO-A1-03/078507
- KR-A- 960 016 869
- KR-A- 20030 074 072
- KR-B1- 101 307 704
- KR-B1- 101 340 274
- KR-B1- 101 502 026
- US-A- 5 814 311

## Description

### [Technical Field]

The present invention relates to a method for preparing a pressed make-up powder with improved moldability and feeling of use.

### [Background Art]

The pressed powder (pressed type powder) made by compression molding of powder particles forms a make-up cosmetic product group such as a powder pact and a tween pact. Unlike the loosed type powder, the pressed powder requires stability in a molded state. In other words, the pressed powder is designed to withstand a constant physical impact abnormality in a compressed state, since it should be free of cracks due to external impacts. Also, in the case of the pressed powder, the amount taken into the make-up tools such as a puff should be constant as an amount suitable for application, and when tapping the pressed powder by the puff, there should be no powder scattering.

The common pressed powder composition is a mixture of cosmetic powder and oil binder (liquid binder), and is produced as a pressed powder by crushing, compression and molding of these components.

In this case, a cosmetic powder requires compression moldability. In general, the compression moldability varies depending on the type of powder, and also varies depending on the coating type and size of the powder.

Among the plate-shaped powders used in the common pressed powder, talc has no elasticity and almost all of these types exhibit moldable property. Also, in the case of titanium dioxide, it has a cohesive property and thus a high bonding force, thereby showing good moldability in itself.

In comparison with this, in the case of nylon, polymethyl methacrylate (PMMA) and silica which are globular powders, it is difficult to perform the compression molding due to the pores between the powder particles. Accordingly, in the case of the common pressed powder composition, it is based on plate-shaped powder, but it is common to mix no more than 20 wt.% of globular powder for spreadability when applying it. If the globular powder is contained in a large amount of 20 wt.% or more, it is impossible to perform compression molding or it is difficult to ensure the durability of the produced product.

The oil binder serves to increase the compression moldability by imparting cohesive force between the powder powders and is used to prevent cracking when the pressed powder is subjected to an external impact and to reduce the powder scattering. However, there is a disadvantage that the oil binder gives a heavy feeling when applied and thus degrades the feeling of use of the pressed powder.

Meanwhile, liquid foundation type products, which are gaining popularity among consumers in recent years, contain polyol or oil to form a sticky cosmetic film, when applied to the skin, in order to make it shiny and glossy. If the conventional pressed powder as described above is coated on such sticky cosmetic film, there is a problem that since the spreadability is reduced and the aggregation phenomenon appears, then uniform application is difficult.

Therefore, it is required to develop a pressed powder with improved spreadability and uniform applicability, and various studies related to it have been conducted. As an example, Korean Patent No. 1502026 discloses a solid powder cosmetic composition which ensures compression stability and moldability by using particulate polymer powders having high cohesiveness to increase compression density, even while not including the oil binder.

On this wise, in order to improve the spreadability and uniform applicability of the pressed powder, there is a need to develop a pressed powder in which the use of the oil binder is reduced and in which the content of the globular powder is increased, but there has not yet been proposed a preparing technique of a pressed powder exhibiting the above-mentioned effect while securing the compression moldability.

US 5 814 311 A discloses a cosmetic composition in the form of a compact powder comprising a fatty phase and a pulverulent phase, said pulverulent phase comprising a first incompatible filler and at least a second filler which may be compactible or incompatible, wherein these two fillers being of different types.

WO 03/078507 A1 discloses a spherical powder component comprising: (1) a large spherical powder having a particle size of from about 10µm to about 50µm; and (2) a small spherical powder having a particle size of from about 1µm to about 10µm; wherein at least one of the large spherical powder and at least one of the small spherical powder are made of the same material, and the weight ratio of the large spherical powder to the small spherical powder is from about 25:1 to about 1:25.

### [Prior Art Literature]

Korean Patent Publication No. 1502026, Oil-free compression or solid powder cosmetic composition.

### [Disclosure]

### [Technical Problem]

The inventors of the present invention have made a study on a method for preparing a pressed powder exhibiting excellent applicability and have completed the present invention.

Therefore, it is an object of the present invention to provide a method for preparing an improved pressed make-up powder containing a high content of globular powder, with improved compression moldability and feeling of use.

### [Technical Solution]

In order to achieve the above object, the present invention provides a method of preparing a pressed make-up powder containing a high content of globular powder by using freeze drying. It is characterized in that since the pressed make-up powder applied to the present invention is prepared by a freeze-drying process, even when a large amount of globular powder is contained, it can be molded while maintaining the shape as it is, and it is possible to omit the powder component and components other than water (for example, binder or thickener).

More particularly, the present invention provides a method for preparing a pressed make-up powder without using oil binder by mixing raw materials containing a globular powder and water to prepare a wet paste, followed by drying it, wherein the drying is performed by freeze drying and the globular powder is contained in an amount of 30 to 60 wt.% of the total weight of the pressed make-up powder as defined in claim 1.

At this time, the particle diameter of the globular powder may be 5 to 20*µ*m. The globular powder is at least one selected from the group consisting of polymethyl methacrylate, polymethylsilsesquioxane, Hdi/trimethylolhexyl lactone crosspolymer, polymethyl methacrylate-Hdi/trimethylolhexyl lactone crosspolymer, ethylene-acrylic acid co-polymer, nylon 12, and silica which are coated or not coated on their surface with hydrophilic or lipophilic coating material. The hydrophilic or lipophilic coating material may be at least one selected from the group consisting of alkylsilane, dimethicone, methicone, trisiloxane, fluorine, and lauroyl lysine.

At this time, the pressed make-up powder may further contain a plate-shaped powder.

At this time, the plate-shaped powder may be at least one selected from the group consisting of talc, mica, sericite, barium sulfate, boron nitride, and alumina. The pressed make-up powder contains a water-soluble binder in an amount of no more than 5 wt.% of the total weight of the pressed make-up powder. The water-soluble binder is at least one selected from the group consisting of magnesium aluminum silicate, sodium magnesium silicate, disteardimonium hectorite, bentonite, hydroxyethylcellulose and cellulose gum.

At this time, the pressed make-up powder may additionally contain a powder binder including at least one selected from the group consisting of polyethylene; stearates including aluminum stearate, calcium stearate, zinc stearate and magnesium stearate; and magnesium myristate.

At this time, the powder binder may be contained in an amount of 1 to 10 wt.% of the total weight of the pressed make-up powder.

At this time, the freeze drying may be performed at -70 to 5°C.

In addition, the present invention provides a pressed make-up powder produced by the above process.

At this time, the pressed make-up powder can be made into a stick type.

The embodiments of the present invention are reflected on independent claims 1 and 9. The preferred embodiments of the present invention are reflected on dependent claims 2 to 8.

### [Advantageous Effects]

According to the pressed make-up powder preparing method of the present invention, it is possible to produce the pressed powder containing a large amount of the globular powder without using oil binder and prevent the breakage of the active ingredients which are unstable in the external environment.

The pressed powder produced according to the present invention contains air in the inner voids generated during the production process and a large amount of globular powder to prevent clumping, provide even coverage, and manifest in a smooth and light feeling of use.

### [Description of Drawings]

FIG. 1 is an image of the conventional formulation (Comparative example 1) formed by compressing a conventional powder.
FIG. 2 is an image of the conventional formulation (Comparative example 2) formed by hot-air drying a wet powder paste.
FIGs. 3 to 5 are images of formulations (Examples 1 to 3) formed by freeze-drying a wet powder paste according to the present invention
FIG. 6 is an image of the conventional pressed powder formulation (Comparative example 1) applied to the surface of the artificial leather.
FIG. 7 is an image of the pressed powder formulation (Example 1) applied to the surface of the artificial leather, which is prepared by freeze-drying according to the present invention.
FIG. 8 is an image of the pressed stick-type powder prepared by the preparation method according to the present invention.

### [Best Mode]

The present invention relates to a method for preparing a pressed make-up powder with the improved moldability and feeling of use by freeze drying process.

In particular, the present invention provides a method for preparing a pressed make-up powder without using an oil binder by mixing raw materials containing a globular powder and water to prepare a wet paste, followed by drying it, wherein the drying is performed by freeze drying and the globular powder is contained in an amount of 30 to 60 wt.% of the total weight of the pressed make-up powder.

The freeze drying is a process by which the solid (ice) water can sublimate into gas (water vapor) by freezing the subject matter and then lowering the partial pressure of the water vapor pressure (below the triple point of water, i.e. 6 mbar or 4.6 Torr or less), which is commonly used as a means for the stable preservation or transport of food.

Since the ice crystals sublimed during freeze drying leave spaces, the freeze-dried material contains a lot of voids. The present inventors have completed the present invention in consideration of this point.

In other words, the pressed powder prepared by freeze drying contains numerous pores generated from sublimation of water inside, and thus shows smooth and light feeling of use when applied to the skin.

In addition, when the wet paste prepared by mixing raw materials is freeze dried, there are advantages that since only the water is sublimed while maintaining the appearance as it is and thus it is possible to perform the compression molding even when a large amount of globular powder is contained, it is possible to produce the pressed powder, and since separate oil binder is not required, the feeling of use of the pressed powder can be improved.

In the present invention, the performing condition of the freeze drying is not particularly limited, but the temperature is preferably -70 to 5°C, the chamber pressure is preferably maintained at 0.1 mbar or less and the drying time is preferably 6 to 24 hours.

The present invention has a significance that the freeze drying, a process of drying the food as described above, was introduced into the process of molding the pressed make-up powder. Some examples of using the freeze-drying process are known in the art, but they are used for the purpose of improving the stability by preventing breakage of the active ingredients contained in the cosmetic. These uses differ from the purpose of enhancing the moldability and the feeling of use of the pressed make-up powder of the present invention.

Hereinafter, the components contained in the pressed make-up powder prepared by the freeze-drying process of the present invention will be described in detail. Further, in addition to the components described below, it is to be noted that there is no limit to the addition of ordinary components within the technical scope of the present invention.

### Globular powder

The globular powder of the present invention is at least one selected from the group consisting of polymethyl methacrylate(PMMA), polymethylsilsesquioxane(PMSQ), Hdi/trimethylolhexyl lactone crosspolymer, polymethyl methacrylate-Hdi/trimethylolhexyl lactone crosspolymer, ethylene-acrylic acid copolymer, nylon 12, and silica which are coated or not coated on their surface with a hydrophilic or lipophilic coating material.

The content of the globular powder contained in the pressed make-up powder according to the present invention is 30 to 60 wt.% based on the total weight of the pressed make-up powder formulation. The reason is that if the content of the globular powder is less than 30 wt.%, an aggregation phenomenon occurs when applied to the skin, the uniformity is not good, and if the content exceeds 60 wt.%, the powder scattering phenomenon occurs and the feeling of use is lowered.

At this time, the particle diameter of the globular powder is preferably 5 to 20*µ*m. If the particle diameter is less than 5*µ*m, it is difficult to provide the smooth feeling of use due to the globular powder, and if the particle diameter exceeds 20*µ*m, it is difficult to perform the compression molding, and there may be a problem with the stability of the formulation, such as the breakage of the formulation at the time of the drop.

The globular powder applicable to the present invention may be any one of hydrophilic or lipophilic non-coating powder, hydrophilic coating powder, lipophilic coating powder and the mixtures thereof. At this time, it is desirable to use a mixture of hydrophilic powder and lipophilic powder for uniform spreadability. In addition, if a coating powder is included, the dispersibility and persistence of the pressed powder can be further improved.

In one embodiment of the present invention, the hydrophilic or lyophophilic material that can be coated on the globular powder may be, but is not limited to, at least one selected from the group consisting of alkylsilane, dimethicone, methicone, trisiloxane, fluorine, and lauroyl lysine, and preferably may be dimethicone or methicone, and can be prepared according to the conventional coating process in the art by a wet or dry coating process.

### Plate-shaped powder

The pressed make-up powder produced according to the present invention may further contain a plate-shaped powder in addition to the above-described globular powder.

The plate-shaped powder may be, but is not limited to, at least one selected from the group consisting of talc, mica, sericite, barium sulfate, boron nitride, and alumina.

The content of the plate-shaped powder can determine the amount of the powder to be applied to the skin with a makeup tool, such as a puff, at the time of one application. In the present invention, the content of the plate-shaped powder is preferably 30 to 70 wt.% based on the weight of the pressed make-up powder formulation. At this time, it is preferable that the plate-shaped powder components except for talc are suitably blended according to the purpose and type of the cosmetic formulation within the range of 1 to 40 wt.%.

### Water-soluble binder

The paste of the pressed make-up powder of the present invention further contains a water-soluble binder.

The water-soluble binder is used to increase the bonding force between powder particles in the aqueous phase using water as a solvent and to facilitate the kneading of the paste and is at least one selected from the group consisting of magnesium aluminum silicate, sodium magnesium silicate, disteardimonium hectorite, bentonite, hydroxyethylcellulose and cellulose gum.

The water-soluble binder is contained in an amount of 5 wt.% or less, preferably 0.1 to 5 wt.%, based on the weight of the pressed make-up powder formulation.

In addition, if the globular powder coated with the silicone-based material such as alkylsilane, dimethicone, methicone, or trisiloxane is used, since the globular powder has difficulty in absorbing water and adsorbing a water-soluble binder, it is not easy to produce a paste. Therefore, it is preferable to use a hydroxyethylcellulose-based binder which thickens water to form a gel so that water does not directly contact the surface of the globular powder

### Powder binder

The paste of the pressed make-up powder of the present invention may further comprise a powder binder along with the water-soluble binder.

Thus, if the powder binder is included, the hardness of the composition becomes higher, and the pressed powder can be implemented as a formulation such as stick-type.

The powder binder is not particularly limited as long as it is commonly used in the cosmetic field, but for example may be any powders classified as viscosity/hardness increasing agents, such as polyethylene, stearates (aluminum, calcium, zinc, magnesium), magnesium myristate. Among them, it is preferable to include at least one selected from the group consisting of magnesium myristate, calcium stearate, and zinc stearate.

The content of the powder binder is not particularly limited but is preferably contained in an amount of 1 to 10 wt.%, more preferably 3 to 5 wt.%, based on the total weight of the pressed make-up powder.

If the paste of the pressed make-up powder of the present invention contains the powder binder, it can be formulated into a stick type which is easy to carry and apply. The stick-type pressed powder can be directly applied to the skin without using a brush or a puff, and thus has advantage of being very easy to use and simply portable.

If the pressed make-up powder produced according to the present invention is embodied as a stick type, the hardness of the paste of the pressed powder containing the water-soluble binder and the powder binder is preferably 30 (N) or more. The hardness can be measured at room temperature (25°C) using Sun rheometer compac-100, CR-500DX, Spin No. 2 (spherical ball).

The reason is that if the hardness of the paste is less than 30 (N), the hardness after freeze drying becomes low, and thus the powder stick is broken and destructed, which makes it difficult to maintain the shape of the stick.

Meanwhile, the paste of the pressed make-up powder according to the present invention may further include a pigment for complementing skin defects and imparting a bright color feeling. The kind of such a pigment is not particularly limited, and a white or colored inorganic or organic particle commonly used in the art can be used.

The inorganic pigments may be, for example, titanium dioxide, zirconium oxide or cerium oxide, or zinc oxide, iron oxide (black, yellow or red), chromium oxide, manganese violet, ultramarine blue, chromium hydroxide hydrate and ferric blue, aluminum powder and copper powder which are surface-treated or not surface-treated.

The organic pigments may be, for example, cochineal extract pigment, azo dyes, anthraquinone dyes, indigoid dyes, xanthene dyes, pyrene dyes, quinoline dyes, triphenylmethane dyes, or fluoran dyes.

In addition, the paste of the pressed powder may further contain at least one of oily components, moisturizer, softener, surfactant, organic and inorganic pigment, organic powder, ultraviolet absorber, antiseptic, thickener, disinfectant, antioxidant, plant extract, pH adjuster, alcohol, pigment, fragrance, blood circulation promoter, cold feeling agent, and adiaphoretic.

Hereinafter, the present invention will be described in more detail by way of specific examples.

The freeze drying applied to the present invention is preferably carried out at -70 to 5°C for 6 to 24 hours while maintaining the pressure of the chamber at 0.1 mbar or less, and the remaining conditions and the manufacturing environment may correspond to a conventional freeze drying process in the art.

### Preparation example 1: Preparation of pact-type pressed powder

**Table 1:**

| Item | | Comparati ve example 1 | Comparati ve example 2 | Example 1 | Example 2 | Example 3 |
|---|---|---|---|---|---|---|
| Globu lar powde r | Polymethyl methacryla te (PMMA) | 20 | 20 | 20 | 25 | 20 |
| | Polymethyl silsesquio xane (PMSQ) | 15 | 15 | 15 | 25 | 15 |
| Oil binde r | Dimethicon e | 5 | - | - | - | - |
| Plate - shape d powde r | Mica (white mica) | 10 | 10 | 10 | 10 | 10 |
| | Talc | To 100 | To 100 | To 100 | To 100 | To 100 |
| Other s | Pearl | 3 | 3 | 3 | 3 | 3 |
| | Preservati ve | 0.3 | 0.3 | 0.3 | 0.3 | 0.3 |
| Total | | 100 | 100 | 100 | 100 | 100 |
| Water - solub le | Magnesium aluminum silicate | - | 3 | 3 | 3 | - |
| | Cellulose | | - | - | - | 3 |
| binde r | gum | | | | | |
| | Purified water | - | 97 | 97 | 97 | 97 |
| Total | | - | 100 | 100 | 100 | 100 |

### Examples 1 to 3

As a pressed powder made by freeze drying, a pact was prepared by the following method according to the composition shown in Table 1.
1) The globular powder, the plate-shaped powder and other ingredients were weighed into the mixer and mixed for 2 minutes.
2) The aqueous solution containing the water-soluble binder was put into a mixer and mixed for 10 minutes to prepare a paste.
3) The paste of the above 2) was freeze-dried under the condition of -70°C in a chamber, and then freeze-dried for 12 hours under conditions of 0.004 mbar and -20 to 5 °C to obtain a pact.

### Comparative example 1

As a conventional pressed powder, a pact was prepared by the following method according to the composition shown in Table 1.
1) The globular powder, the plate-shaped powder and other ingredients were weighed into the mixer and mixed for 2 minutes.
2) The oil binder components were weighed and heated to 70∼80°C.
3) The above 2) was sprayed and then mixed and disintegrated for 1 to 2 minutes using a Henschel mixer (500 to 1000 rpm).
4) The above 3) was mixed at a high speed (1500 to 2000 rpm) for 2 minutes.
5) The above 4) was crushed at 50 horsepowers and then filtered.
6) A certain amount of the mixture was placed on a Petri dish and then molded at about 200 kgf/cm for about 5 seconds to prepare a pact.

### Comparative example 2

As a pressed powder made by hot air drying, a pact was prepared by the following method according to the composition shown in Table 1.
1) The globular powder, the plate-shaped powder and other ingredients were weighed into the mixer and mixed for 2 minutes.
2) The water-soluble binder was weighed into the above 1), and the mixture was mixed at a high speed (1500 to 2000 rpm) for 10 minutes to prepare a paste.
3) The paste of the above 2) was hot-air dried at 55°C for 12 hours in a chamber to prepare a pact.

### Experimental example 1: Appearance evaluation

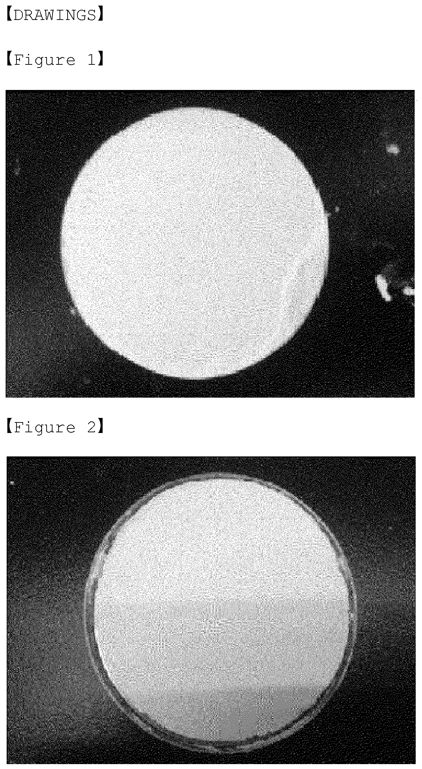

FIGs. 1 to 5 are appearance photographs of the pact-type pressed powders of Comparative examples 1 and 2 and Examples 1 to 3 prepared in Preparation example 1, respectively

In Comparative example 1 produced by a conventional pressed powder preparation method, it was confirmed that since the globular powder is contained in a high content, the molding is not performed well, and the contents of the powder are released from the dish as shown in FIG. 1. Also, in Comparative example 2 prepared by hot air drying, the contents were shrunk and separated from the dish as shown in FIG. 2.

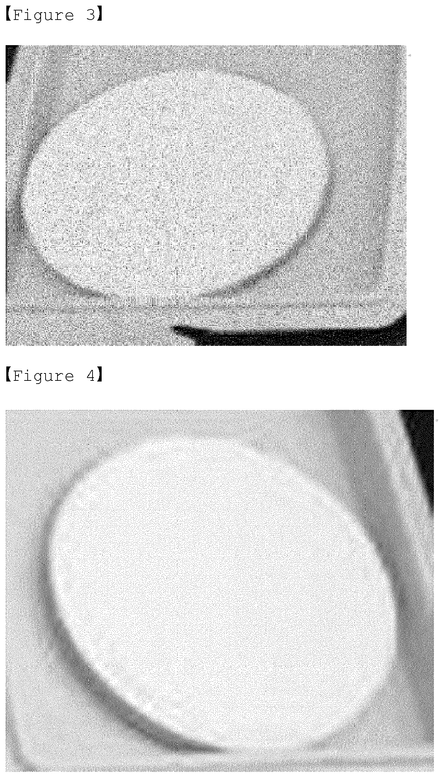

On the other hand, it can be seen that Examples 1 to 3 prepared by freeze drying contain a high content of globular powder but are dried while maintaining the shape as it is immediately after filling as shown in FIGs. 3 to 5.

### Experimental example 2: Measurement of residual powder weight after drying

12.0 g of each of the pressed powder compositions of Comparative Examples 1 and 2 and Example 1 prepared according to composition of Table 1 above was taken and filled in a Petri dish and then compressed at a high pressure or dried to measure the residual powder weight. The results are shown in Table 2 below.

**Table 2:**

| Test item | Comparative example 1 | Comparative example 2 | Example 1 |
|---|---|---|---|
| Residual powder weight (g) | 12.0 | 7.45 | 7.43 |

In Comparative example 1, there was no difference in weight before and after filling when measuring the weight including the weight of the contents to be removed, and in both of the comparative example 2 and example 1, since the moisture was dried from the contents and the powder was left only, the weight was decreased.

### Experimental example 3: Sensory evaluation

The pacts of Comparative Example 1, Comparative Example 2 and Example 1 were applied to the skin of 20 professional panelists from the early 20s to the mid 40s for one month, and the sensory evaluation was carried out on lightness, uniformity, softness and powder scattering. The results are shown in Table 3 below (⊚: Very excellent, ○: Excellent, Δ: Fair, ×: poor).

**Table 3:**

| Evaluation item | Comparative example 1 | Comparative example 2 | Example 1 |
|---|---|---|---|
| Lightness | Δ | ○ | ⊚ |
| Uniform applicability | × | ○ | ⊚ |
| Softness | Δ | × | ⊚ |
| No powder scattering | × | × | ○ |

As a result of evaluation, the pact of Example 1 by freeze drying was found to be generally superior in all items. In the case of Example 1, since the water is sublimated and there is air in the remaining pore area, the powder formulation is evaluated as being light. Also, in the case of Example 1, it was found to be smooth and uniform, and to be lightly applied, and to exhibit less powder scattering when applied, and thus it was found that the pressed powder prepared according to the present invention is very excellent in terms of the feeling of use.

### Experimental example 4: Applicability evaluation

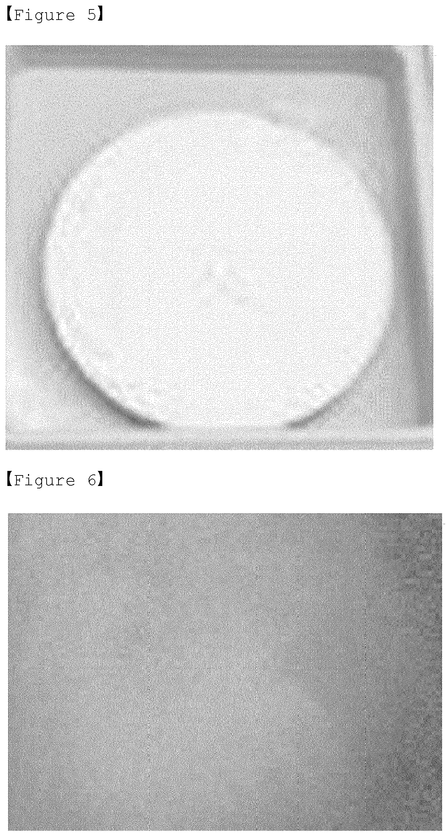

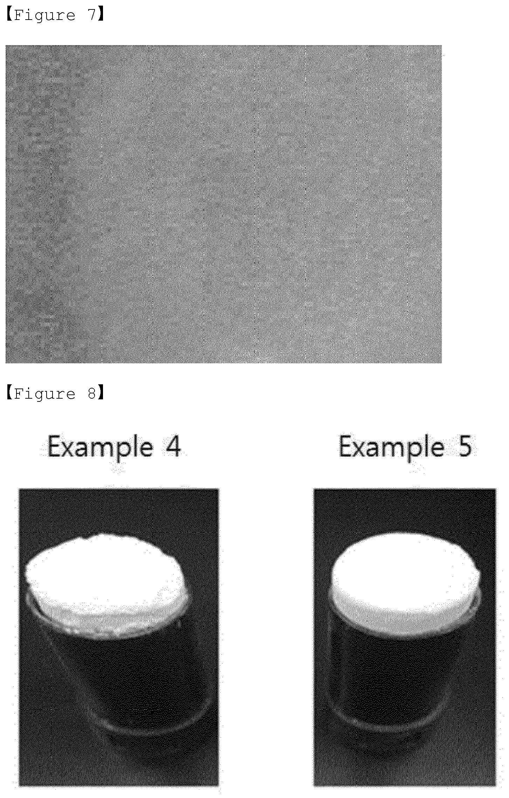

A visual test was performed to evaluate the uniformity when applied. FIG. 6 shows the result of taking 0.1 g of the formulation of the above-mentioned Comparative Example 1 into the puff and then tapping it three times onto the surface of the black artificial leather, and FIG. 7 shows the results obtained by taking 0.1 g of the formulation of Example 1 above into the puff and tapping it three times onto the surface of the black artificial leather. Referring to FIG. 6, the formulation of Comparative Example 1 is not uniformly applied when applied once, and aggregation phenomenon occurs even when it is tapped only twice or three times. On the other hand, referring to FIG. 7, it was found that the formulation of Example 1 is applied in an appropriate amount and spread uniformly without aggregation.

### Preparation example 2: Preparation of stick-type pressed powder

**Table 4:**

| Item | | Example 4 | Example 5 |
|---|---|---|---|
| Globular powder | Polymethyl methacrylate (PMMA) | 20 | 20 |
| | Polymethylsilsesquioxane (PMSQ) | 15 | 15 |
| Plate-shaped powder | Mica (white mica) | 10 | 10 |
| | Talc | To 100 | To 100 |
| Others | Pearl | 3 | 3 |
| | magnesium myristate | - | 3 |
| | Preservative | 0.3 | 0.3 |
| Total | | 100 | 100 |
| Water-soluble binder | Magnesium aluminum silicate | 3 | 1 |
| | Purified water | 97 | 99 |
| Total | | 100 | 100 |

### Examples 4 and 5

The stick-type pressed powder was prepared using the same preparation method as in Examples 1 to 3 with the composition shown in Table 4 above.

### Experimental example 5: Evaluation of appearance and feeling of use

The appearance and the feeling of use of the stick-type pressed powder prepared in Preparation example 2 was evaluated. At this time, the evaluation of the feeling of use was performed in the same manner as Experimental example 3, and the results are shown in Table 5 below.

**Table 5:**

| Evaluation item | Example 4 | Example 5 |
|---|---|---|
| Application convenience (hardness) | Δ | ○ |
| Uniform applicability | × | ○ |
| Softness | ○ | ○ |
| No powder scattering | × | ○ |

As shown in FIG. 8, the stick of Example 4 had a low hardness and thus showed a crushing phenomenon, and as a result of evaluation of the feeling of use, showed some powder scattering when applied. This is thought to be due to the fact that the powder binder was not contained and thus the hardness sufficient to be made into a stick type was not ensured.

On the other hand, the stick of Example 5 with the powder binder added had a sufficient hardness, and thus did not show a crushing phenomenon, and exhibited a smooth surface. In addition, the stick of Example 5 was evaluated as having no powder scattering and being excellent in softness and uniformity when applied.

The pressed make-up powder according to the present invention can be formulated into two-way cake, pact, stick, blusher, eye shadow, highlighter, and concealer.

## Claims

1. A method for preparing a pressed make-up powder without using oil binder, by mixing raw materials containing a globular powder and water to prepare a wet paste, followed by drying it,
wherein the drying is performed by freeze drying, and wherein the globular powder is contained in an amount of 30 to 60 wt.% of the total weight of the pressed make-up powder; and
wherein the globular powder is at least one selected from the group consisting of polymethyl methacrylate, polymethylsilsesquioxane, Hdi/trimethylolhexyl lactone crosspolymer, polymethyl methacrylate-Hdi/trimethylolhexyl lactone crosspolymer, ethylene-acrylic acid copolymer, nylon 12, and silica which are coated or not coated on their surface with a hydrophilic or lipophilic coating material; and
wherein the pressed make-up powder further contains a water-soluble binder in an amount of 5 wt.% or less of the total weight of the pressed make-up powder; and
wherein the water-soluble binder is at least one selected from the group consisting of magnesium aluminum silicate, sodium magnesium silicate, disteardimonium hectorite, bentonite, hydroxyethylcellulose and cellulose gum.

2. The method for preparing the pressed make-up powder according to claim 1, **characterized in that** the particle diameter of the globular powder is 5 to 20*µ*m.

3. The method for preparing the pressed make-up powder according to claim 1, **characterized in that** the hydrophilic or lipophilic coating material is at least one selected from the group consisting of alkylsilane, dimethicone, methicone, trisiloxane, fluorine, and lauroyl lysine.

4. The method for preparing the pressed make-up powder according to claim 1, **characterized in that** the pressed make-up powder further contains a plate-shaped powder.

5. The method for preparing the pressed make-up powder according to claim 4, **characterized in that** the plate-shaped powder is at least one selected from the group consisting of talc, mica, sericite, barium sulfate, boron nitride and alumina.

6. The method for preparing the pressed make-up powder according to claim 1, **characterized in that** the pressed make-up powder further contains a powder binder including at least one selected from the group consisting of polyethylenes; stearates including aluminium stearate, calcium stearate, zinc stearate and magnesium stearate; and magnesium myristate.

7. The method for preparing the pressed make-up powder according to claim 6, **characterized in that** the powder binder is contained in an amount of 1 to 10 wt.% of the total weight of the pressed make-up powder.

8. The method for preparing the pressed make-up powder according to claim 1, **characterized in that** the freeze drying is carried out at -70 to 5°C.

9. A pressed make-up powder **characterized by** being produced by any one of the methods of claims 1 to 8.

## Patentansprüche

1. Verfahren zur Herstellung eines gepressten Make-up-Pulvers ohne Verwendung von Ölbindemittel durch Mischen von Rohstoffen, die ein globuläres Pulver und Wasser enthalten, zur Herstellung einer feuchten Paste und anschließendes Trocknen derselben;
wobei das Trocknen durch Gefriertrocknen erfolgt und wobei das globuläre Pulver in einer Menge von 30 bis 60 Gew.-% des Gesamtgewichts des gepressten Make-up-Pulvers enthalten ist; und
wobei das globuläre Pulver wenigstens eines ist, das aus der Gruppe ausgewählt ist, die aus Polymethylmethacrylat, Polymethylsilsesquioxan, Hdi/Trimethylolhexyllacton-Kreuzpolymer, Polymethylmethacrylat-Hdi/Trimethylolhexyllacton-Kreuzpolymer, Ethylen-Acrylsäure-Copolymer, Nylon 12 und Siliciumoxid besteht, die auf ihrer Oberfläche mit einem hydrophilen oder lipophilen Beschichtungsmaterial beschichtet sind oder auch nicht; und
wobei das gepresste Make-up-Pulver weiterhin ein wasserlösliches Bindemittel in einer Menge von 5 Gew.-% oder weniger des Gesamtgewichts des gepressten Make-up-Pulvers enthält; und
wobei das wasserlösliche Bindemittel wenigstens eines ist, das aus der Gruppe ausgewählt ist, die aus Magnesiumaluminiumsilicat, Natriummagnesiumsilicat, Disteardimoniumhectorit, Bentonit, Hydroxyethylcellulose und Cellulosegummi besteht.

2. Verfahren zur Herstellung des gepressten Make-up-Pulvers gemäß Anspruch 1, **dadurch gekennzeichnet, dass** der Teilchendurchmesser des globulären Pulvers 5 bis 20 µm beträgt.

3. Verfahren zur Herstellung des gepressten Make-up-Pulvers gemäß Anspruch 1, **dadurch gekennzeichnet, dass** das hydrophile oder lipophile Beschichtungsmaterial wenigstens eines ist, das aus der Gruppe ausgewählt ist, die aus Alkylsilan, Dimethicon, Methicon, Trisiloxan, Fluor und Lauroyllysin besteht.

4. Verfahren zur Herstellung des gepressten Make-up-Pulvers gemäß Anspruch 1, **dadurch gekennzeichnet, dass** das gepresste Make-up-Pulver weiterhin ein plättchenförmiges Pulver enthält.

5. Verfahren zur Herstellung des gepressten Make-up-Pulvers gemäß Anspruch 4, **dadurch gekennzeichnet, dass** das plättchenförmige Pulver wenigstens eines ist, das aus der Gruppe ausgewählt ist, die aus Talk, Glimmer, Sericit, Bariumsulfat, Bornitrid und Aluminiumoxid besteht.

6. Verfahren zur Herstellung des gepressten Make-up-Pulvers gemäß Anspruch 1, **dadurch gekennzeichnet, dass** das gepresste Make-up-Pulver weiterhin ein Pulverbindemittel enthält, das wenigstens eines umfasst, das aus der Gruppe ausgewählt ist, die aus Polyethylenen, Stearaten einschließlich Aluminiumstearat, Calciumstearat, Zinkstearat und Magnesiumstearat sowie Magnesiummyristat besteht.

7. Verfahren zur Herstellung des gepressten Make-up-Pulvers gemäß Anspruch 6, **dadurch gekennzeichnet, dass** das Pulverbindemittel in einer Menge von 1 bis 10 Gew.-% des Gesamtgewichts des gepressten Make-up-Pulvers enthalten ist.

8. Verfahren zur Herstellung des gepressten Make-up-Pulvers gemäß Anspruch 1, **dadurch gekennzeichnet, dass** das Gefriertrocknen bei -70 bis 5 °C durchgeführt wird.

9. Gepresstes Make-up-Pulver, **dadurch gekennzeichnet, dass** es nach einem der Verfahren gemäß Anspruch 1 bis 8 hergestellt ist.

## Revendications

1. Procédé pour préparer une poudre de maquillage comprimée sans utiliser un liant huileux, en mélangeant des matières premières contenant une poudre globulaire et de l'eau pour préparer une pâte humide, suivi d'un séchage,
dans lequel le séchage est effectué par lyophilisation, et dans lequel la poudre globulaire est contenue en une quantité de 30 à 60 % en poids, par rapport au poids total de la poudre de maquillage comprimée, et
dans lequel la poudre globulaire est au moins une choisie dans le groupe consistant en poly(méthacrylate de méthyle), polyméthylsilsesquioxane, polymère croisé de Hdi/lactone de triméthylolhexyle, polymère croisé de poly(méthacrylate de méthyle)-Hdi/lactone de triméthylolhexyle, copolymère d'éthylène/acide acrylique, nylon 12, et silice, qui sont enrobés ou non avec un matériau d'enrobage hydrophile ou lipophile sur leur surface, et
dans lequel la poudre de maquillage comprimée contient en outre un liant soluble dans l'eau, en une quantité de 5 % en poids ou moins, par rapport au poids total de la poudre de maquillage comprimée, et
dans lequel le liant soluble dans l'eau est au moins un choisi dans le groupe consistant en silicate de magnésium-aluminium, silicate de sodium-magnésium, hectorite de distéardimonium, bentonite, hydroxyéthylcellulose, et gomme de cellulose.

2. Procédé pour préparer la poudre de maquillage comprimée selon la revendication 1, **caractérisé en ce que** le diamètre de particule de la poudre globulaire est de 5 à 20 µm.

3. Procédé pour préparer la poudre de maquillage comprimée selon la revendication 1, **caractérisé en ce que** le matériau d'enrobage hydrophile ou lipophile est au moins un choisi dans le groupe consistant en alkylsilane, diméthicone, méthicone, trisiloxane, fluoré, et lauroyl-lysine.

4. Procédé pour préparer la poudre de maquillage comprimée selon la revendication 1, **caractérisé en ce que** la poudre de maquillage comprimée contient en outre une poudre en forme de paillettes.

5. Procédé pour préparer la poudre de maquillage comprimée selon la revendication 4, **caractérisé en ce que** ladite poudre en forme de paillettes est au moins une choisie dans le groupe consistant en talc, mica, séricite, sulfate de baryum, nitrure de bore, et alumine.

6. Procédé pour préparer la poudre de maquillage comprimée selon la revendication 1, **caractérisé en ce que** la poudre de maquillage comprimée contient en outre un liant de poudre incluant au moins un choisi dans le groupe consistant en polyéthylènes, stéarates incluant le stéarate d'aluminium, le stéarate de calcium, le stéarate de zinc, et le stéarate de magnésium, et le myristate de magnésium.

7. Procédé pour préparer la poudre de maquillage comprimée selon la revendication 6, **caractérisé en ce que** ledit liant de poudre est contenu en une quantité de 1 à 10 % en poids, par rapport au poids total de la poudre de maquillage comprimée.

8. Procédé pour préparer la poudre de maquillage comprimée selon la revendication 1, **caractérisé en ce que** la lyophilisation est effectuée à -70 à 5 °C.

9. Poudre de maquillage comprimée, **caractérisée en ce qu'**elle a été produite par l'un quelconque des procédés selon les revendications 1 à 8.
